# EUROPEAN PATENT APPLICATION

(11) **EP 1 801 594 A1**
(43) Date of publication of application: **27.06.2007**
(21) Application number: 05112924.5
(22) Date of filing: 23.12.2005
(51) Int. Cl.: G01N 33/68, G01N 33/543, G01N 33/58, G01N 35/00, C07K 16/42

(54) **Sensing device and method for determination of the amount of target molecule in an analyte**

(71) Applicant: Koninklijke Philips Electronics N.V., 5621 BA Eindhoven (NL)
(72) Inventor: The designation of the inventor has not yet been filed
(74) Representative: Schmitz, Herman Jan Renier

(57) **Abstract**

A sensing device is provided which has a universal sensor surface. This allows easy modification of the detection properties without the need for investigation of the binding properties of a new target to the sensor surface. The device comprises a sensor surface, which has attached thereto a primary capture molecule. The invention further relates to a method for measurement of a target wherein this device is used.

## Description

### Field of the invention

The present invention relates to a method for detecting target molecules in an analyte composition and to a sensing device suitable for use in this method. The invention especially relates to a method wherein use is made of magnetic labels and a suitable magnetic sensor device.

### Background to the invention

In the field of diagnostics, especially in biomedical diagnostics, such as medical and food diagnostics the use of biosensors or biochips is well known. These biosensors and chips are generally used in the form of micro-arrays of biochips, enabling the analysis of target molecules such as biological molecules e.g. DNA, RNA, proteins, enzymes, hormones, drugs. Many types of assays and detection systems are used in the currently available sensors. Generally labels are used for detection. Examples of labels are optical labels, coloured beads, fluorescent chemical groups, barcodes or magnetic labels.

For example GB-A-2404022 discloses the detection of methamphetamine class of drugs such as ecstasy. This document discloses competitive assays, which comprise
a) Contacting a sample comprising the drug to be identified with (i) a pseudoephedrine/carrier conjugate in which pseudoephedrine is linked via its hydroxyl group to the carrier and (ii) an antibody which is capable of binding both one or more drugs of the methamphetamine group and said conjugate; and
b) Determining whether the binding of said antibody to said conjugate is reduced by the presence of said sample, a reduction in binding being indicative that the sample contains a methamphetamine group drug. The antibody may comprise a detectable label such as a detectable enzyme like horseradish peroxidase.

One of the disadvantages of this method is that the target molecule or a molecule with the same binding properties (a pseudo-target), requires binding to the carrier. Hence for each new target that is to be analyzed, a different molecule requires immobilization on a carrier. The binding process is a complicated process that may differ significantly for other targets or pseudo-targets. This is a major drawback in the development of universal sensors.

Therefore it is an object of the current invention to provide a method and device which enable the use of universal sensor surfaces without the need to modify the sensor surface for each new target that is to be analyzed.

This drawback specifically arises when use is made of magnetic sensors. Because of the underlying mechanisms of detecting a change in magnetic field, in such sensors it is desired that a magnetic label is bound to a sensor surface. This is only possible either directly or indirectly by providing a link to the sensor surface via other molecules. It is desired that the sensor surface is a universal sensor surface, which can be used for the analysis of a variety of targets. This would obviate the need to modify the surface each time a new target is measured.

The current invention overcomes at least some of these drawbacks.

### Summary of the invention

It has surprisingly been found that if instead of immobilizing the analyte or pseudo-target on a sensor surface, the sensor surface is covered with analyte-binding molecules, this leads to more universal sensor surfaces.

Therefore the invention in one aspect relates to a method for detecting target molecules using a sensing device, wherein the method comprises
a) Providing a sensing device for determining the concentration of at least one type of target in an analyte composition,
   the sensing device comprising at least one sensing surface,
   the surface having attached thereto at least one primary capture molecule which is an antibody or is a molecule capable of binding to an antibody,
   the sensing device further comprising a sensing element,
b) Contacting the analyte composition with the sensing surface
c) Optionally providing in fluid form a secondary target molecule that specifically binds the target molecule
d) Providing a composition comprising labeled anti-idiotype antibody
e) Detecting the signal provided by the label.

In a further aspect the invention relates to a sensing device suitable for use in this method and to a system and kit for use in this method.

### Detailed description of the invention

### Description of the figures

Figure 1 shows an embodiment of the invention where an antibody specific for the target molecule is immobilized on a sensor surface.
Figure 2 shows an embodiment where a universal capture molecule is attached to the sensor surface.

Where an indefmite or definite article is used when referring to a singular noun, e.g. "a", "an", "the", this includes a plural of that noun unless something else is specifically stated.

It is to be noticed that the term "comprising" used in the present description and claims, should not be interpreted as restricted to the means listed thereafter; it does not exclude other elements or steps.

In the context of the invention the term "antibody" refers to antibodies and their functional derivates such as Fab fragments, ScFv, and heavy chain only antibodies.

A "capture molecule" is a molecule that binds another molecule. Examples of capture molecules are affibodies, antibodies, receptor molecules, aptamers and chelators.

Anti-idiotype antibody is defined as an antibody or other binding molecule directed against the antigen specific part of the sequence of an antibody or T-cell receptor and which thus recognises the antigen binding sites of other antibodies or T-cell receptors. Generally anti-idiotype antibodies are specific for a class of antibodies or for the antibodies that originate from a single species. Examples of suitable other binding molecules are affibodies, and DNA aptamers. It is preferred to use anti-idiotype antibody.

In the context of the invention, antibody that specifically binds to a target is an antibody which binds more strongly or preferentially to the target molecule in a competitive assay but shows little or no cross reactivity with other molecules.

A capture molecule with broad specificity or binding affinity for a broad range of antibodies that specifically bind a target is for example selected from protein A and protein G that bind to the Fc part of an antibody.

In a first aspect the invention relates to a method for detecting target molecules using a sensing device, wherein the method comprises a first step (a) of providing a sensing device for determining the concentration of at least one type of target in an analyte composition. This sensing device comprises at least one sensing surface, the surface having attached thereto at least one primary capture molecule which is an antibody or is a molecule capable of binding to an antibody. An analyte composition, which comprises the target which is to be measured, is brought into contact with the surface. This may for example be carried out by injecting fluid comprising the analyte (or the analyte if it is already in a suitable fluid form), into the sensing device such that it comes into contact with the sensor surface. In the embodiment where the primary capture molecule is an antibody, the antibody will bind the target provided the conditions such as temperature and salt concentration of the fluid are suitable for binding. Simultaneously or in a separate next step, a composition comprising labeled anti-idiotype antibody is contacted with the sensor surface. In those places where there is no target bound, the anti-idiotype antibody will bind to the target-specific antibody that is attached to the surface of the sensor. The signal that is provided by the label may be detected by the sensor.

In the embodiment where the primary capture molecule is a molecule that is capable of binding antibodies, a secondary capture molecule that specifically binds the target molecule is provided after or during the contacting of the analyte composition with the sensor surface. In a further step, which takes place after this or simultaneously, labeled anti-idiotype antibody is contacted with the sensor surface. In this embodiment, it is preferred that the primary capture molecule is saturated with secondary capture molecule. The primary capture molecule in this embodiment, preferably binds to a broad range of antibodies that are capable of specific binding to target. This "universal capture molecule" preferably has low affinity for the target.

The primary capture molecule may be linked to the sensor surface in any suitable way. Below we will describe that preferably specific parts are attached to the sensor surface. This attaching (also referred to as linking, coating or bonding) may be by any suitable method such as covalent linking or non-covalent linking. It is preferred that the attaching is via directed interaction rather than by random binding. An example of a link is via a sulfur bridge when an available cysteine residue is present on the primary capture molecule.

In a preferred embodiment, after contacting the analyte with the sensing surface, a washing step is included to remove non-specifically bound molecules.

The sensor may be based on any suitable detection mechanism using a label, which is for example selected from fluorescent labels, radioactive labels and magnetic labels. The label may be directly linked to the anti-idiotype antibody or alternatively, detection may occur after the label group is exposed and coupled to another label such as a magnetic molecule or a fluorescent molecule, a magnetic label being preferred. The coupling with the other label may e.g. be via a ligand/binder interaction, such as biotin-streptavidin or a hapten/anti-hapten interaction such as digoxigenin/anti-digoxigenin.

This method was found to be specifically suitable for the detection of molecules using a magnetic sensor and a magnetic label. Magnetic sensors are generally made to be as sensitive as possible to magnetic labels (beads) that are bound to the sensor surface directly or indirectly.

In a preferred embodiment the sensing device is a magnetic sensor and the label is a magnetic label.

In an alternative embodiment the label of the anti-idiotype antibody is non-magnetic, and the method further comprises the step of exposing the anti-idiotype moiety to a magnetic entity.

In the set up of the current method, the label is linked directly or indirectly to an anti-idiotype antibody such that the level of the signal that is caused by the label is inversely related to the amount of target molecule that is present in the analyte. Preferably the method comprises the additional steps of determining the amount of labeled molecules bound to the sensing surface, and
determining the amount of target based on the amount of the labeled molecules.

The label is preferably covalently linked to an anti-idiotype antibody. In a further aspect the invention relates to an antibody-label conjugate comprising an anti-idiotype antibody that is covalently linked to a magnetic label.

To provide a conjugate of a magnetic label and an anti-idiotype antibody, the surface of the magnetic label may be modified. This modification can be done, for example, through covering the surface of the magnetic label with dextrane, alkanethiols with suitable end groups, certain peptides etc. A dextrane molecule may covalently bind to another molecule such as the antibody through cyanobromide activation or carboxylic acid activation.

The invention provides a method or device wherein a universal sensor surface is present. This enables the easy and quick adaptation of the method for new targets. In one embodiment of the invention the sensing surface has attached thereto an antibody that specifically binds target molecule.

If an antibody is attached to the sensor surface it is preferred that the antibody is attached to the sensing surface via the constant domain. This way only the variable domain needs adaptation if the sensor is made suitable for use with another target. In this way the binding characteristics of the antibody to the surface can be easily transferred to the new antibody. The binding to the surface is done in the same way for a variety of antibodies.

Although it will be still be necessary to provide new antibody with another variable domain, it is an important simplification if the surface-binding chemistry can stay the same.

Optionally a linker is included between the surface and the primary capture molecule.

In the alternative embodiment the sensing surface has attached thereto a universal primary capture molecule which can bind to a broad range of molecules and wherein the method further comprises the step of providing in fluid form a secondary capture molecule that specifically binds the target molecule. The primary capture molecule is such that it binds the secondary capture molecule.

In a preferred embodiment, the sensing surface has attached thereto a primary capture molecule that binds a broad range of target specific antibodies.
To make the sensor even more universal, it is preferred that the sensing surface has attached thereto a primary capture molecule which binds to the constant domain of target specific antibodies.

If the method uses magnetic detection, it is preferred that the sensor further comprises a magneto-resistive sensor element embedded in the sensing surface.

If the label is a magnetic label, use is generally made of magnetic particles which may have a size between 10 nm and a few micrometers, more preferred between 30 nm and 300 nm. In a preferred method the magnetic particle is larger than the individual biological molecules involved in the assay.

Magnetic particles may be actuated by magnetic fields. When forces are applied in such a way that the magnetic particles are brought to the sensor surface, the biological binding rate can be enhanced.

The magnetic labels may be any shape or form. The labels include any form of one ore more magnetic particles e.g. magnetic, diamagnetic, paramagnetic, superparamagnetic, ferromagnetic, that is any form of magnetism which generates a magnetic dipole in an magnetic field, either permanently or temporarily.

In the method of the invention all reagents may be brought into contact with each other and the sensor surface at once (e.g. in one reaction chamber) or they may be brought into contact sequentially in time. To keep control over the binding processes it preferred that steps a-e are carried out sequentially. Optionally washing steps are included in between the steps.

In a further aspect the invention relates to a sensing device for determining the concentration of a target molecule in an analyte composition via determination of the concentration of a label,
- the sensing device comprising at least one sensing surface
- the sensing surface having attached thereto at least one primary capture molecule which is an antibody or a molecule capable of binding to an antibody,
- the sensing device further comprising at least one sensor element, preferably a magnetic sensor element,
characterized in that the primary capture molecule comprises a constant domain and a binding domain and in that the primary capture molecule is attached to the sensing surface via the constant domain.

Preferably the capture molecule is an antibody specifically binding the target, which antibody molecule is attached to the sensing surface via the constant domain.

Even more preferred, the sensing surface has attached thereto a capture molecule that binds to the constant domain of target specific antibodies.

The proposed method and sensor are suitable for use with small reaction chambers and small sensor surfaces in the micro to nano range. This allows for easy sensor multiplexing into a larger system to provide a system, which can carry out several assays simultaneously on a single chip or cartridge.

Therefore the invention also relates to a system for determining the concentration of at least one type of target, the system comprising the sensing device as described above.

It is preferred that the sensing device is a magnetic sensor. In the magnetic sensor, a magnetic sensor element is included which may be selected from the group comprising an AMR^{(A)}, a GMR^{(B)}, or a TMR^{(C)} sensor element. Of course, magnetic sensor elements based on other principles like Hall sensor elements or SQUIDS are also possible for use in the device according to the invention.
A: Anomalous Magnetoresistive
B: Giant Magnetoresistive:
C: Tunnel Magnetoresistive

The sensor surface may be made of any suitable surface composition. Examples of preferred surface materials are organic or inorganic material e.g. glass, plastic, silicon, or a combination of these.

The sensor further comprises magnetic field generating means. These magnetic field generating means may, for example, be magnetic materials (rotating or nonrotating), and/or conductors such as current wires.

In a preferred embodiment, the invention relates to a magnetic sensor comprising
i. Magnetic field generating means
ii. An inlet for sample introduction
iii. A sensor surface having attached thereto a primary capture molecule wherein the primary capture molecule comprises a constant domain and a binding domain and in that the primary capture molecule is attached to the sensing surface via the constant domain.
iv. An outlet for fluid
v. A magnetic sensor element

In another aspect the invention relates to a kit of parts comprising a composition comprising anti-idiotype antibody having attached thereto a magnetic label, and a separate composition comprising antibody that specifically binds target material.

Preferably part of the kit is a sensor device comprising a surface comprising immoblized thereon an antibody that specifically binds target material.

In a further embodiment the invention relates to a kit of parts or sensing device as described above, wherein, when present, capture molecule and/or anti idiotype antibody are present in dried form. This enables storage of the kit or device for a long time without reducing the binding properties of the compositions. This dried form may be obtained in any suitable way, e.g. by drying in air or optionally applying a vacuum.

In use, the addition of fluids will enable the resuspension of the dried material such that it can have its normal binding function again.

In another embodiment the invention relates to a sensing device comprising a sensing surface having attached thereto a primary capture molecule in dried form.

Optionally in the embodiment where the primary capture molecule is a capture molecule with broad specificity for binding to other antibodies, this capture molecule is present in the kit or sensing device, attached to a surface, in dry form, and the antibody that specifically binds to a target is present in dry form as well. The target specific antibody may e.g. be present in a reservoir or a channel that is connected to the surface, and may be brought in the vicinity of the surface once fluid is forced through such a channel or reservoir.

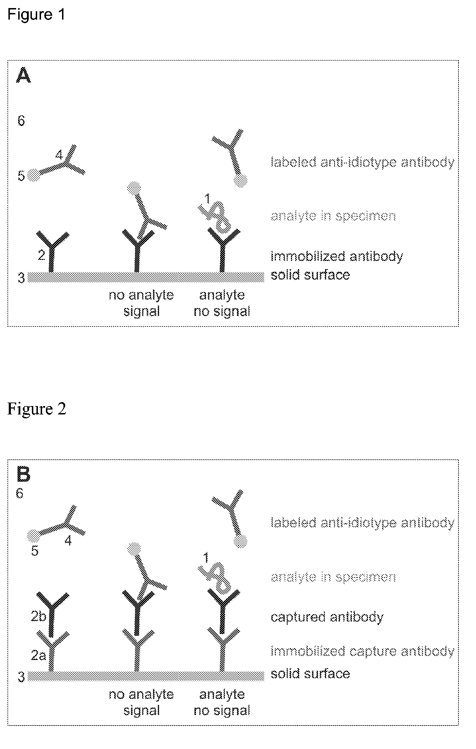

Figures 1 and 2 illustrate two specific embodiments of the invention.

Figure 1 shows a composition comprising target compound 1 of which the presence and concentration is to be determined. In this embodiment an antibody specific for the target is immobilized on the sensor surface 3. When a fluid sample 6 contains the target, it will bind to the immobilized antibody. If no target is present, an anti-idiotype molecule 4 (in this case an anti-idiotype antibody) that is labeled with a detection moiety 5 will bind to the immobilized antibody instead. The binding of this anti-idiotype antibody to the immobilized specific antibody will give rise to a signal. The level of the signal is inversely related to the amount of target that is present in the composition that is analyzed. The specific antibody is preferably bound to the surface via its constant domain. The sensor can be made suitable for another target by modifying the binding part of the antibody. The way the constant part of the antibody is bound to the surface can stay the same, which provides enhanced standardization.

In figure 2 a universal primary capture molecule 2a is attached to the sensor surface. This molecule captures the target-specific antibody 2b. Molecule 2a can for example be a ligand-receptor (in case ligand is coupled to 2b), an anti-hapten antibody (in case a hapten is coupled to 2a) or a secondary antibody (directed against the Fc part of 2a in case 2a is an unmodified antibody). A composition comprising the target is brought into contact with the sensor surface. The target is bound by target specific antibody (2b). Where no target is bound, in a further step, a labeled anti-idiotype antibody binds the target-specific antibody. Bound target-specific antibody directly or indirectly causes a signal. In this method, the level of the signal is inversely related to the amount of target. The sensor can be made suitable for the analysis of a different target by inclusion of another target specific antibody.

In an example situation the sensor is made initially for target (i) and adapted to measure target (ii). The adaptation can be accomplished by removal of the target (i) specific antibody by application of dissociation conditions. These dissociation conditions are preferably such that the universal primary capture molecule (2a) remains bound to the sensor surface. Following the dissociation and removal of target (i) specific antibody, the target (ii) specific antibody is brought into contact with the sensor surface and bound to the universal capture molecule under association conditions. The set up of the sensor and assay method are unchanged for the measurement of target (ii). This is an example of enhanced standardization of the sensor.

In another example that benefits from the enhanced standardization of the surface an array of sensors is integrated in a single chip that has a uniform surface over the entire chip. Different types of receptor molecules need to be bound to this uniform surface above each of the sensor elements in the integrated array. In this way a sensor can be made that can simultaneously detect target (i) and target (ii).

## Claims

1. A method for detecting target molecules using a sensing device, wherein the method comprises
a). Providing a sensing device for determining the concentration of at least one type of target in an analyte composition,
the sensing device comprising at least one sensing surface,
the surface having attached thereto at least one primary capture molecule which is an antibody or is a molecule capable of binding to an antibody,
the sensing device further comprising a sensing element,
b). Contacting the analyte composition with the sensing surface
c). Optionally providing in fluid form a secondary capture molecule that specifically binds the target molecule
d). Providing a composition comprising labeled anti-idiotype antibody
e.) Detecting the signal provided by the label.

2. A method according to claim 1 wherein the sensing device is a magnetic sensor and the label is a magnetic label.

3. A method according to claim 1 wherein the level of the signal is inversely related to the amount of target molecule that is present in the analyte which method comprises the additional steps of determining the amount of labeled molecules bound to the sensing surface, and
determining the amount of target to be measured based on the amount of the labeled molecules.

4. A method according to claim 1 wherein sensing surface has attached thereto an antibody that specifically binds target molecule.

5. A method according to claim 4 wherein the antibody is attached to the sensing surface via the constant domain.

6. A method according to claim 2 wherein the sensor further comprises a magneto-resistive sensor element embedded in the sensing surface.

7. A method according to claim 1 wherein the label of the anti-idiotype antibody is non-magnetic, which methods further comprises the step of exposing the anti-idiotype moiety to a magnetic entity.

8. A method according to claim 1 wherein the sensing surface has attached thereto a universal capture molecule which can bind to a broad range of molecules and wherein the method further comprises the step of providing in fluid form a secondary molecule that specifically binds the target molecule.

9. A method according to claim 8 wherein the sensing surface has attached thereto a capture molecule that binds a broad range of target specific antibodies.

10. A method according to claim 9 wherein the sensing surface has attached thereto a capture molecule, that binds to the constant domain of target specific antibodies.

11. Sensing device for determining the concentration of a target molecule in an analyte composition via determination of the concentration of a label,
the sensing device comprising at least one sensing surface
the sensing surface having attached thereto at least one primary capture molecule which is an antibody or a molecule capable of binding to an antibody,
the sensing device further comprising at least one sensor element,
**characterized in that** the capture molecule comprises a constant domain and a binding domain and **in that** the primary capture molecule is attached to the sensing surface via the constant domain.

12. Sensing device according to claim 11 wherein the sensor element is a magnetic sensor element.

13. Sensing device according to claim 11 wherein the primary capture molecule is an antibody specifically binding the target, which is attached to the sensing surface via the constant domain.

14. Sensing device according to claim 11 wherein the sensing surface has attached thereto a primary capture molecule, that binds to the constant domain of target specific antibodies.

15. Magnetic sensor comprising
i. Magnetic field generating means
ii. An inlet for sample introduction
iii. A sensor surface having attached thereto a primary capture molecule wherein the capture molecule comprises a constant domain and a binding domain and in that the primary capture molecule is attached to the sensing surface via the constant domain.
iv. An outlet for fluid
v. A magnetic sensor element

16. Kit of parts comprising a fluid composition comprising anti-idiotype antibody having attached thereto a magnetic label, and a separate composition comprising antibody that specifically binds target material.

17. Kit of parts according to claim 16 wherein antibody that specifically binds target material is immobilized on a surface.

18. System for determining the concentration of at least one type of target, the system comprising the sensing device according to any of claims 11-14.

19. Antibody-label conjugate comprising an anti-idiotype antibody that is covalently linked to a magnetic label.
